# EUROPEAN PATENT APPLICATION

(11) **EP 4 335 385 A2**
(43) Date of publication of application: **13.03.2024**
(21) Application number: 23193336.7
(22) Date of filing: 24.08.2023
(51) Int. Cl.: A61B 10/00

(54) **MENSTRUAL CYCLE TRACKING USING TEMPERATURE MEASUREMENTS**

(30) Priority: 06.09.2022 US 202263403937 P; 21.07.2023 US 202318224924
(71) Applicant: Apple Inc., Cupertino, CA 95014 (US)
(72) Inventor: ZHANG, Shunan, Cupertino, 95014 (US); CURRY, Christine L., Cupertino, 95014 (US); ZHANG, Carey Y., Cupertino, 95014 (US); PARK, Jihyun, Cupertino, 95014 (US); WANG, Yikai, Cupertino, 95014 (US)
(74) Representative: Zacco Denmark A/S

(57) **Abstract**

Embodiments are directed to systems and methods for tracking menstrual cycles of a user. Embodiments can include obtaining a first set of temperature data at an electronic device, and in response to the first set of temperature data satisfying a first criteria, determining a first probability that ovulation occurred during a first time period using the first set of temperature data. In response to the first probability meeting a second criteria, embodiments can include determining a second set of probabilities comprising a probability that ovulation occurred for each day of a first set of days within the first time period. An estimated ovulation date can be determined using the second set of probabilities, and an electronic device can display an output indicating the estimated ovulation date.

## Description

### CROSS-REFERENCE TO RELATED APPLICATION

### FIELD

The described embodiments relate generally to menstrual cycle tracking and prediction. More particularly, the present embodiments relate to estimating an ovulation date, fertile window, and/or period start date based on biometric information of a user, such as temperature data.

### BACKGROUND

Many people that experience menstrual cycles plan activities around portions of their menstrual cycle, such as a fertile window and/or period. Menstrual cycles can be irregular, leading to an inability to reliably plan activities. Further, attempts to gather data that may be used to estimate or predict portions of a menstrual cycle may be difficult, invasive, and inaccurate

### SUMMARY

Embodiments are directed to methods for tracking a menstrual cycle of a user. The methods can include obtaining a first set of temperature data at an electronic device and determining that the first set of temperature data meets a first criteria. In response to determining that the first set of temperature data meets the first criteria, the methods can include determining a first probability that ovulation occurred during a first set of days using the first set of temperature data. The methods can include determining that the first probability meets a second criteria and in response to determining that the first probability meets the second criteria, determining a set of probabilities. The methods can include selecting an estimated ovulation date from the first set of days using the set of probabilities and displaying an output on the electronic device indicating the estimated ovulation date. The second set of probabilities can include, for each day in the first set of days, a probability that ovulation occurred on the day.

Embodiments are also directed to methods for estimating ovulation of a user. The methods can include determining, at an electronic device, a start date of a first menstrual cycle of the user and obtaining, following the start date of the first menstrual cycle, a set of temperature data at the electronic device. The methods can include determining, using the set of temperature data, a set of probabilities comprising, for each day of a set of days, a corresponding probability that ovulation occurred on the day and determining an estimated ovulation date of the first menstrual cycle using the set of probabilities. The methods can include displaying a first output on the electronic device based the estimated ovulation date, determining a start date of a second menstrual cycle subsequent to the first menstrual cycle, and determining an updated ovulation date of the first menstrual cycle using an updated start date of the second menstrual cycle. The methods can include displaying a second output on the electronic device based on the updated ovulation date.

Embodiments are further directed to an electronic device for tracking menstrual cycles of a user. The electronic device may include one or more temperature sensors that measure temperatures of the user, a display, and a processor configured to collect a set of temperature measurements using the one or more temperature sensors. The processor can be configured to operate in a first mode to determine that a first subset of the set of temperature measurements associated with a first set of days meets a first criteria and use a first set of operations to determine a first estimated ovulation date for the user using the first subset of the set of temperature measurements. The first set of operations can include an artificial neural network that outputs a set of probabilities including a probability that ovulation occurred for each day of a window of days. The processor can be configured in a second mode to determine that a second subset of the set of temperature measurements associated with a second set of days meets a second criteria and use a second set of operations to determine a second estimated ovulation date using the second subset of the set of temperature measurements, a start date of a menstrual cycle, and an end date of the menstrual cycle.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 shows an example graph of temperature data during a menstrual cycle;
FIG. 2 shows a system architecture of an embodiment of a menstrual cycle estimator;
FIG. 3 shows an example process flow for estimating an ovulation date using temperature measurements;
FIGs 4A and 4B show example user interfaces displaying estimated fertility and ovulation information;
FIG. 5 shows an example process flow for updating an estimated ovulation date and/or fertile window based on additional menstrual cycle data;
FIG. 6 shows an example process flow for estimating an ovulation date by identifying temperature shifts using temperature data;
FIG. 7 shows an example wearable device that may be used to track menstrual cycles using temperature measurements; and
FIG. 8 shows example components of the wearable device of FIG. 7.

### DETAILED DESCRIPTION

Reference will now be made in detail to representative embodiments illustrated in the accompanying drawings. It should be understood that the following descriptions are not intended to limit the embodiments to one preferred embodiment. To the contrary, it is intended to cover alternatives, modifications, and equivalents as can be included within the spirit and scope of the described embodiments as defined by the appended claims.

Embodiments described herein generally take the form of techniques for estimating dates of various portions of a menstrual cycle, such as an ovulation date, fertile window, and/or period start date. The techniques track these portions of menstrual cycles using temperature data collected from the user, and in some instances using additional collected data such as heart rate data and/or user input logging the start and stop of a given menstrual cycle. Depending on the amount and types of data available, the mechanism may use different techniques to estimate the timing of portions of the menstrual cycle. Thus, as the mechanisms receive additional data, they may be able to refine these estimations (e.g., a predicted ovulation date, fertile window, and/or period start date).

In some embodiments, the techniques described herein are incorporated into a system that includes one or more temperature sensors that collect temperature data from a user. The system also includes a set of operational modules that perform the various steps described herein. Specifically, the operation modules include a cycle status module, a data processing module, and an ovulation estimation module, which collectively are used to estimate a user's ovulation date (i.e., the day during which ovulation occurred) and/or the user's' fertile window. In some instances, the one or more temperature sensors and the operation modules may be incorporated into a single device, such as a wearable device.

In other instances, the techniques described herein may be performed as a method. In some instances, the method can include receiving an initial estimate of a fertile window for a current menstrual cycle and an initial estimate of a period start date of a subsequent menstrual cycle. The method can include receiving temperature data and determining whether the temperature data meets a data sufficiency criteria that may represent a set of requirements that determines if there is sufficient temperature data to accurately perform an ovulation estimate. In the event the temperature data meets the data sufficiency criteria, the method can include estimating an ovulation date using the temperature data. In response to estimating the ovulation date, a fertile window can be updated and the ovulation date and/or updates to the fertile window can be displayed to and electronically accessible by a user.

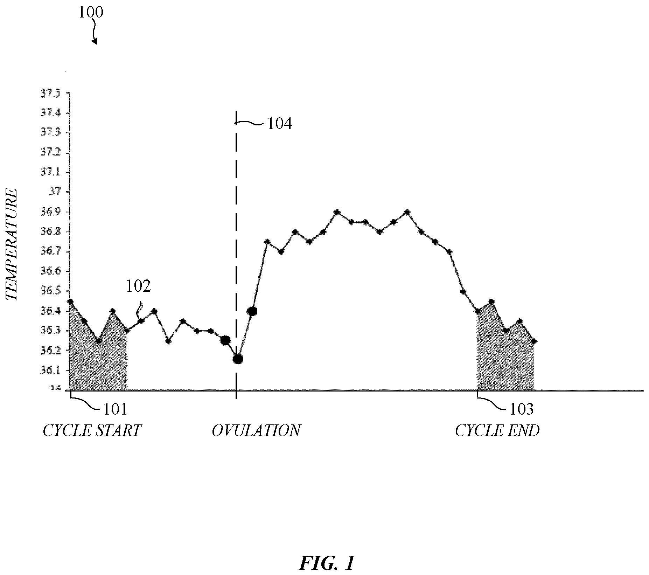

These and other embodiments are discussed below with reference to FIGs. 1-8. However, those skilled in the art will readily appreciate that the detailed description given herein with respect to these Figures is for explanatory purposes only and should not be construed as limiting.

A "menstrual cycle" as used herein lasts from the start of a period to the day before the start of the next period and typically includes a follicular phase, ovulation, and a luteal phase. A "fertile window" is the portion of the menstrual cycle during which a person is most likely to conceive; it typically begins several days (e.g., up to five days) before ovulation and typically ends the day of ovulation. The follicular phase occurs after the period and prior to ovulation and the luteal phase occurs post-ovulation, extending to the start of the next period. The techniques described herein may be used to estimate dates associated with a user's current menstrual cycle (referred to herein as the "ongoing cycle"). Additionally or alternatively, the techniques described herein may be used to estimate dates associated with a menstrual cycle that has already ended (referred to herein as a "historical cycle") or predict one or more dates of a menstrual cycle that has yet to begin (referred to herein as a "future cycle").

Menstrual cycles are tracked from a "day one" instantiation, which is the first day that a user initiates tracking by marking a first day of menstrual bleeding (i.e., the "period start date"). On day one, the next period is predicted for a user, which represents an estimate of the start of the next future cycle (as well as the end of the ongoing cycle). The fertile window and/or ovulation date for the ongoing cycle may also be estimated. Within the user's ongoing cycle, data collected by the systems, devices, and methods described herein may allow for an estimation of whether ovulation has occurred. Accordingly, embodiments of the systems, devices, and methods described herein can update the initial predictions of the ovulation date, fertile window, and/or next period start date mid-cycle based on obtained data.

Specifically, embodiments of the systems, devices, and methods described herein are configured to track a user's temperature over multiple days and analyze this temperature data to estimate the timing of various portions of a menstrual cycle. A user's body temperature, in particular the user's basal body temperature (or "BBT", which is the body's temperature while at rest), will change over the course of an ongoing menstrual cycle. For example, a user's BBT will typically increase in response to ovulation and remain elevated during the luteal phase until the start of the next period. Thus, detecting an increase in the body temperature of a user may indicate that the user has ovulated. By detecting the ovulation date of a given menstrual cycle, the systems, devices, and methods may update the initial estimates of the ovulation date, fertile window or, in the instance of an ongoing cycle, next period start date.

FIG. 1 shows an example graph of a user's temperature during a menstrual cycle 100. For the purpose of illustration, each day is represented by a single temperature value (e.g., a measurement of the user's BBT). Each menstrual cycle 100 starts on the day the period begins 101 and ends when the next period begins 103. Temperatures 102 during each menstrual cycle 100 typically exhibit a biphasic pattern, where they are lower prior to ovulation 104 and higher after ovulation 104. As shown in FIG. 1, the user's body temperature 102 typically increases at ovulation 104 and remains elevated until the start of the next menstrual cycle approaches. While the specific temperature values shown in FIG. 1 are just an example (and do not reflect temperature values of any particular person), most people have a similar correlation between body temperature and various portions of the menstrual cycle. This may be especially true if the user's body temperature is gathered and averaged, smoothed, or otherwise algorithmically correlated across multiple menstrual cycles. Thus, a user's body temperature may be used to estimate various portions of a menstrual cycle, or to refine estimations of such portions of a menstrual cycle, such as the onset and end of a fertile window, a period start date, and/or an ovulation date.

Specifically, the systems, methods, and devices may use one or more techniques to estimate whether this ovulation-based temperature change (referred to herein as an "ovulation temperature shift") has occurred. Identification of an ovulation temperature shift can then be used to estimate an ovulation date for a given menstrual cycle (e.g., the ovulation date 104 of menstrual cycle 100 in FIG. 1). Temperature information from a user may be used during an ongoing cycle to identify an ovulation temperature shift (and thereby the ovulation date) during the ongoing cycle or may be used to retroactively identify an ovulation temperature shift (and thereby the ovulation date) in a historical cycle. When retroactively estimating an ovulation date in a historical cycle, additional information such as an end date of the menstrual cycle (e.g., which may be determined when a user inputs a period that designates the start of a new menstrual cycle) may be used in performing the retroactive estimation.

In some instances, the estimated ovulation date for a given menstrual cycle may be used in estimating or predicting other aspects of that menstrual cycle or subsequent menstrual cycles. For example, the estimated ovulation date 104 determined for an ongoing menstrual cycle 100 can be used to calculate a fertile window for that menstrual cycle. Additionally or alternatively, the estimated ovulation date 104 may be used to predict the start date of a subsequent menstrual cycle and/or an ovulation date of the subsequent menstrual cycle. In some cases, menstrual cycle start dates and/or ovulation can be tracked over multiple menstrual cycles 100, and this combined data can be used to predict menstrual events such as a period start date and/or an ovulation date for subsequent menstrual cycles 100.

As mentioned above, the devices, systems, and methods described herein involve collecting temperature data from a user using one or more temperature sensors. The temperature data may be collected by any suitable number or type of temperature sensors, which may be split across multiple separate devices. In instances where temperature data is collected from multiple temperature sensors, it may be desirable to calculate an adjustment for each temperature sensor to account for sensor-specific deviations in temperature readings. For example, two temperature sensors measuring the same object may yield slightly different measurements and adjusting for these differences may improve the accuracy of the ovulation date estimation techniques described herein. In other instances, the devices, systems, and methods described herein may utilize temperature data from a single temperature sensor (or multiple temperature sensors from a single device) for the purpose of estimating an ovulation date, even if temperature measurements from additional sensors and/or devices are available.

The devices, systems, and methods described herein may optionally collect additional user information that may used in estimating an ovulation date, a fertility window, and/or next period start as described herein. In some instances, an input received by a user (e.g., manually or in response to a prompt provided by one of the devices described herein) may be used to indicate the start date of a period, which indicates the end of one menstrual cycle and the start of a new one. The user may enter (or the systems and devices described herein may be able to automatically determine) additional information, such as user demographics (e.g., their age, body measurements), food and/or alcohol intake, current medications, sleep information, or the like.

Additionally or alternatively, the devices, systems, and methods described herein may receive additional biometric data from one or more other sensors, such as heart rate information (e.g., a user's heart rate, resting heart rate, heart rate variability, or the like), respiration rate, blood pressure, blood oxygenation, or the like. These additional sensors may be incorporated into the same device that includes a temperature sensor (or sensors) as discussed above or may be part of a separate device from the one or more temperature sensors. For example, an embodiment may take the form of a portable, wearable device that has one or more temperature sensors. This wearable device may further include one or more additional sensors, such as a photoplethysmography sensor, configured to measure and collect this additional biometric data.

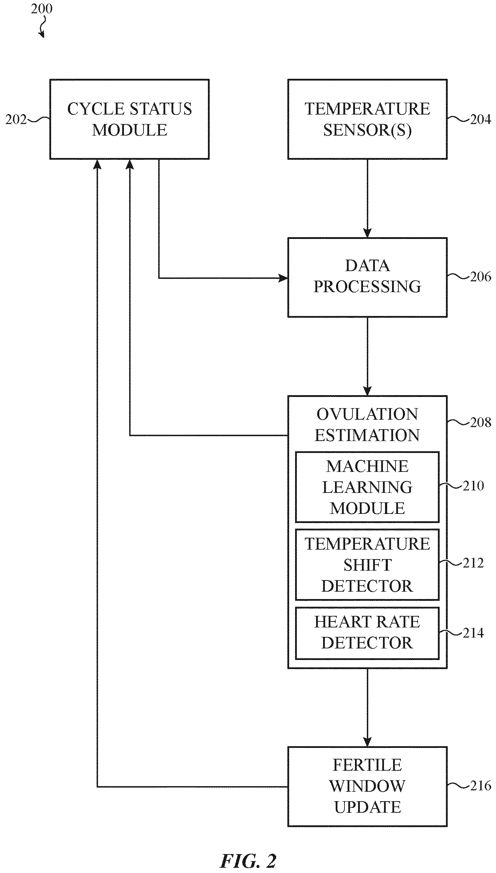

FIG. 2 shows a system architecture 200 of an embodiment of a system as described herein that is configured to estimate an ovulation date. Specifically, FIG. 2 generally illustrates example operational modules that may perform the various techniques described herein. Accordingly, any of the operations performed by each operational module described herein may be stored as instructions on a non-transitory computer-readable storage device, such that a processor may utilize these instructions to perform these operations. Similarly, the systems and devices described herein include a memory and one or more processors operatively coupled to the memory. The one or more processors may receive instructions from the memory and are configured to execute these instructions to perform the various operations of each operational module. The system architecture 200 is configured to estimate an ovulation date of a given menstrual cycle and may further estimate the fertile window for that menstrual cycle and/or the period start date for a subsequent menstrual cycle.

The operational modules of the system architecture 200 cooperate to perform these estimations. For example, the operation modules may include a cycle status module 202, a data processing module 206, an ovulation estimation module 208, and a fertile window update module 216. The cycle status module 202 maintains information relating to a user's menstrual cycles. The cycle status module 202 may contain user-inputted data and/or estimates relating to one or more historical cycles, the user's ongoing cycle, and/or one or more future cycles. For example, for a given historical cycle, the cycle status module 202 may store data that includes a period start date, a period end date, an ovulation date, and a fertile window. Depending on the cycle, some or all of this data may be entered by a user or may be estimated by the system architecture 200 when the user input is unavailable. Similarly, the ongoing cycle may include a period start date, a period end date, an ovulation date (which, depending on how far along in the ongoing cycle a user is, may be an estimate of when ovulation has occurred or an estimate of when ovulation will occur), and a fertile window. Because a future cycle necessarily has not started (e.g., a period start date marks a new ongoing cycle), the cycle status module may maintain estimated predictions of the period start date, period end date, an ovulation date, and a fertile window.

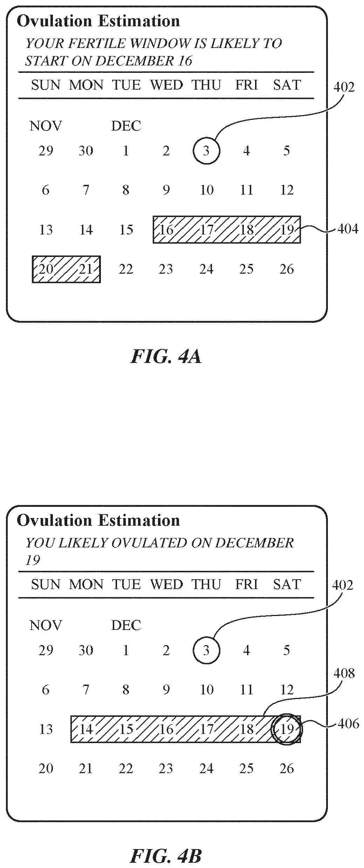

Information from the cycle status module 202 may be used to generate a user interface that depicts information about the user's menstrual cycle, such as discussed below with respect to FIGS. 4A and 4B. These user interfaces may be generated on demand from a user, or in response to certain predetermined criteria.

In certain embodiments, at the start of an ongoing cycle the cycle status module 202 may provide an initial (or "day zero") estimate of either or both of a fertile window and the period of the next menstrual cycle. More specifically, the cycle status module 202 may provide a range of dates on which the next period may start and end, classifying some of those dates as possible start or end dates and others as more likely start or end dates. The cycle status module 202 may do the same for the fertile window, although in some embodiments the calendar may not present less likely dates for the fertile window to a user. For future cycles, the calendar may provide an estimate of the first day of a menstrual cycle, a fertile window within the menstrual cycle, and an estimate of a next period start date. This information may be provided to a user through a calendar or other application executed by a computing device so that the user may plan future activities accordingly. This information may be used to plan attempts to become pregnant or, conversely, for contraception, for travelling, and so on.

The system architecture also includes temperature sensor(s) 204, which may include a single temperature sensor or multiple temperature sensors as described previously. The temperature sensor(s) can periodically measure a user's body temperature to obtain a set of temperature measurements. Individual temperature measurements may be taken on demand (i.e., initiated by a user) or opportunistically, such as at predefined intervals when a given temperature sensor is worn by the user. Each temperature measurement may include a temperature value and associated additional information, such as the time of day (e.g., day vs. night) the measurement was taken, a designation of a user's sleep state (e.g., asleep vs. awake, or a designation of a particular sleep stage), a quality metric (e.g., an amount of device or user motion associated with the temperature measurement), combinations thereof, and the like. This associated additional information may facilitate the system architecture in selecting different subsets of these temperature measurements for analysis using the techniques described herein.

The data processing module 206 may receive the calendar data and temperature data and place it in a form to be used by the ovulation estimation module 208. Specifically, the data processing module 206 may select a subset of the temperature measurements and generate a set of temperature data using the subset of temperature measurements. Accordingly, when the devices, systems, and methods described herein discuss "obtaining" data or measurements (e.g., temperature data or heart rate data), this includes selecting a subset of available measurements. In some instances, the obtained data may be generated from the selected subset of available measurements.

As mentioned previously, the system architecture 200 may use different techniques to estimate an ovulation date depending on certain characteristics of the temperature measurements received by the temperature sensor(s) 204. Specifically, the ovulation estimation module 208 may operate in multiple different modes that each use a different technique to estimate an ovulation date for a given menstrual cycle, and each mode may be associated with a different data sufficiency criteria. For modes that use temperature data to estimate an ovulation date, the data sufficiency criteria may be based on having a minimum number of temperature measurements that meet certain criteria. For example, a data sufficiency criteria may require that a predetermined number of days within a window of time (e.g., twelve days out of the preceding fourteen days) that each has a minimum number of temperature measurements, or a minimum number of temperature measurements meeting a certain criteria (e.g., temperature measurements taken overnight while a user is sleeping, or the like).

The data processing module 206 may determine, for each mode and its associated ovulation estimation technique, whether a subset of temperature measurements meets the corresponding data sufficiency criteria. If the data sufficiency criteria is met for a given mode, the data processing module 206 may generate temperature data from the subset of temperature measurements, which may be used by the ovulation estimation module 208 as described below. The temperature data may include some or all of the subset of temperature measurements or may be derived from some or all of the subset of temperature measurements. Because the number of temperature measurements collected may vary over time, it may be desirable for the data processing module 206 to generate the temperature data in a common form that is utilized by the ovulation estimation module 208.

In some instances, the data processing module 206 determines that there are not sufficient temperature measurements for the ovulation estimation module 208 to operate using modes that utilize temperature data. In these instances, the data processing module 206 may determine whether a data sufficiency criteria is met for a mode that uses heart rate data. In these instances, the data sufficiency criteria may be based on having a minimum number of heart rate measurements that meet certain criteria.

The ovulation estimation module 208 may operate to estimate an ovulation date of a given menstrual cycle and may further be used to estimate a fertile window for that menstrual cycle, or the period start date of a subsequent menstrual cycle. The ovulation estimation module 208 may attempt to estimate the ovulation date for a menstrual cycle at one or more predetermined times. For example, for an ongoing cycle, the ovulation estimation module 208 may attempt to estimate an ovulation date for the ongoing cycle at predetermined intervals (e.g., once per day). Since temperature measurements may be collected on an ongoing basis, each attempt to estimate an ovulation date may utilize a different set of temperature data, though it should be appreciated that these different sets of temperature data may be selected or generated from some common temperature measurements.

In some of these instances, the ovulation estimation module 208 will not attempt to estimate the ovulation date for the ongoing cycle until a threshold amount of time has elapsed from the start date of the menstrual cycle (e.g., five days), which prevents the ovulation estimation module 208 from prematurely estimating ovulation during certain portions of the menstrual cycle. Similarly, once the ovulation estimation module 208 estimates that ovulation has occurred within the ongoing cycle, the ovulation estimation module 208 may stop attempting to estimate the ovulation date (either immediately, or after a predetermined number of intervals following estimation of the ovulation date). Although the ovulation estimation module 208 may have already estimated that ovulation has occurred within the ongoing cycle, performing additional estimations using the ovulation estimation module 208 with additional temperature data may refine the estimation of the ovulation date, and with it the predicted next period start date. In some variations, when the ongoing cycle finishes (and thus becomes a historical cycle), the ovulation estimation module 208 will perform an additional estimation of the ovulation date. In these instances, because the end date of the menstrual cycle is known (because a new menstrual cycle has started), the end date of the menstrual cycle may be used as an input to help refine the estimation of the ovulation date.

For a given attempt to estimate an ovulation date of a given cycle (e.g., an ongoing cycle or a historical cycle as outlined above), the ovulation estimation module 208 may operate in one or more modes, each of which utilizes a different technique to estimate the ovulation date. The ovulation estimation module 208 may include multiple modules that each use different techniques to estimate menstrual events, and the ovulation estimation module 208 may utilize a different module depending on the mode in which it is operating. The different modules may be selected based on the data that has been collected and/or may be used at different times during a menstrual cycle. For example, some modules may require temperature sensor(s) to be worn for relatively large periods of time over the course of a certain set of days, other modules may be able to operate with intermittent temperature measurements, and/or other modules may not require any temperature measurements and instead may utilize other types of data such as heart rate measurements.

In some instances, for a given attempt to estimate an ovulation date of a given menstrual cycle, the ovulation estimation module 208 may select and operate in a single mode (and corresponding estimation technique). In these instances, the ovulation estimation module 208 may select a technique based on the available temperature measurements. There may be a hierarchy of techniques, such that a first technique (and corresponding module of the ovulation estimation module 208) is used if a first data sufficiency criteria is met. If the first data sufficiency criteria is not met, but a second data sufficiency criteria for a second technique is met, the system architecture 200 may select the second technique. If neither of these criteria is met, but a third data sufficiency criteria associated with a third technique is met, a system architecture 200 may select the third technique.

In other instances, for a given attempt to estimate an ovulation date of a given menstrual cycle, the ovulation estimation module 208 may operate in multiple modes to estimate multiple estimates of the ovulation date. For example, if the data sufficiency criteria for multiple modes are met, the ovulation estimation module 208 may attempt to estimate the ovulation date using each of these modes. In these instances, it may be possible for different modes to make different determinations regarding ovulation (e.g., different estimates may disagree as to whether ovulation occurs, or may estimate different ovulation dates), and the ovulation estimation module 208 may use these determinations (e.g., using confidence information or other weighting factors) to estimate the ovulation date.

Returning to FIG. 2, the ovulation estimation module 208 can include a machine learning module 210 that uses trained data sets and/or other classification and regression algorithms to estimate an ovulation date using the measured temperature data. In general, the machine learning module 210 includes two stages. A first stage estimates whether ovulation has occurred within a set of days (also referred herein as a window of days), and a second stage estimates a probability, for each day within the set of days, that ovulation occurred on that day. These probabilities collectively form a set of probabilities, and the machine learning module 210 selects one of the set of days as the estimated ovulation date. In other cases, the ovulation estimation module 208 may output an estimate ovulation occurrence using additional or alternative time periods. For example, the output may indicate a range of hours or other division of time.

Specifically, for a given ovulation estimation, the machine learning module 210 receives a set of temperature data associated with a first set of days. The first stage uses the set of temperature data to determine whether ovulation has occurred within the first set of days. It should be appreciated that the set of temperature data may include or be generated from a larger set of days than the first set of days. For example, the first stage may determine whether ovulation has occurred in a window of five days (e.g., the five days prior to when the ovulation estimate is attempted), but may utilize temperature data based on (e.g., selected and/or generated from) temperature measurements collected over ten or more days (e.g., the ten days prior to when the ovulation estimate is attempted). The first set of days may be a subset of the days for which data is analyzed. In some instances, the larger set of days may optionally include one or more days from a historical cycle preceding the ongoing cycle.

The first stage may include a classifier component that is configured to receive the temperature data and output a probability that ovulation occurred during the first set of days. The classifier may be trained using temperature data and ground truth ovulation dates. In some cases, the classifier component can include a random forest algorithm. The probability from the first stage may be compared to a probability threshold or other selection criteria to estimate whether ovulation has occurred. The probability threshold may be a static value or may be dynamically determined using information from historical cycles of the user.

In instances where the machine learning module 210 is used to estimate an ovulation date of an ongoing cycle, the machine learning module 210 may be iteratively run until the first stage determines that ovulation has occurred. Specifically, if the data processing module 206 determines that a set of temperature data meets the data sufficiency criteria for the machine learning module 210, the first stage will determine a probability that ovulation occurred during a first set of days using the set of temperature data. If this determined probability does not meet the selection criteria, the machine learning module 210 will cease the current estimation. In response, additional temperature measurements may be collected and the data processing module 206 obtains a second set of temperature data that is associated with a second set of days and meets the data sufficiency criteria. In some instances, heart rate data or other sensor measurements can also be input into the machine learning model. The first stage will determine a probability that ovulation occurred during a second set of days (which may partially overlap the first set of days) using the second set of temperature data and determine whether this probability meets the selection criteria. Additional sets of temperature data associated with additional sets of days may be obtained and analyzed as needed until a result from the model satisfies the threshold criteria. When the first stage does calculate a probability that meets the selection criteria, that set of temperature data is passed to the second stage.

In instances where the machine learning module 210 is used to estimate an ovulation date of a historical cycle, the first stage of machine learning module 210 may be iteratively run across multiple sets of days. The multiple sets of days may be selected based on one or more aspects of the historical cycle (such as the start and/or end date of the cycle). In these instances, each of the multiple sets of days is associated with a corresponding set of temperature data, and the first stage calculates a corresponding probability that ovulation occurred during that set of days. The machine learning module 210 may select the set of days from the multiple sets of days with the highest corresponding probability. The selected set of days (and its corresponding set of temperature data) may then be passed to the second stage.

The second stage receives a set of temperature data associated with a set of days and uses that set of temperature data to determine a set of probabilities as discussed previously. For example, when the probability that ovulation occurred during the second set of days of an ongoing cycle meets the selection criteria as discussed above, the second stage uses the second set of temperature data to determine, for each day in the second set of days, that ovulation occurred on that day. For example, the second stage may include a regression component that estimates a probability that ovulation occurred on each day of the set of days. The ovulation estimation may estimate an ovulation date based on the output from the regression component. In some of these variations, the regression component can include a long short-term memory (LSTM) artificial neural network.

As mentioned previously, the machine learning module 210 may be associated with a corresponding data sufficiency criteria, which may be used to determine whether to use the machine learning module 210 for a given ovulation date estimation. Depending on the configuration of the machine learning module 210, the machine learning module 210 may have a relatively stringent data sufficiency criteria, which may require regular temperature measurements around the ovulation date. In instances where the temperature sensor(s) are incorporated into a wearable device, this may entail the user wearing the wearable device around the ovulation date (e.g., while they sleep).

In addition to or as an alternative to the machine learning module 210, the ovulation estimation module 208 can include a temperature shift detection module 212 that can estimate the ovulation data using a different process and with different data sufficiency criteria as compared to the machine learning module 210. The temperature shift detection module 212 may be configured to detect an increase in temperature indicating ovulation. The temperature shift detection module 212 may use an algorithm that compares temperatures from a first set of days to temperatures from a second set of days to estimate whether ovulation occurred on a particular day.

Specifically, to estimate whether ovulation occurred on a particular day, the temperature shift detection module 212 selects a first number of days preceding the day and a second number of days following the day (though it should be considered that the day itself may be included in either the first number or the second number). The first number of days and the second number of days may have the same number of days or a different number of days as desired. The temperature shift detection module 212 determines one or more temperature metrics (e.g., average temperature, average basal body temperature, or the like) for each of the first number of days and the second number of days and determines whether a difference in the determined temperature metrics meets a difference criteria.

For example, the temperature shift detection module 212 may determine the difference between an average temperature for the first number of days (e.g., the six preceding days) and an average temperature for the second number of days (e.g., the three following days). When the temperature shift detection module 212 is used to estimate ovulation in an ongoing cycle, the temperature shift detection module 212 may determine whether the average temperature of the first number of days is less than the average temperature of the second number of days by a threshold amount (e.g., 0.1 degrees Celsius, 0.2 degrees Celsius). If this average temperature has shifted by the threshold amount, the temperature shift detection module 212 may estimate that ovulation occurred on that date. If the average temperature has not shifted by the threshold amount, the temperature shift detection module 212 may make a subsequent attempt to estimate that ovulation occurred on another day (e.g., as additional temperature measurements are collected).

When the temperature shift detection module 212 is used to estimate ovulation in a historical cycle, the temperature shift detection module 212 may select a window of days. For each day within the window of days, the temperature shift detection module 212 may calculate a corresponding temperature change between an average temperature of a first number of days preceding the day and an average temperature of a second number of days following the day. The temperature shift detection module 212 may select a day from the window of days as the estimated ovulation date using the determined temperature changes. For example, the temperature shift detection module 212 may select the day with the highest determined temperature change as the estimated ovulation date.

The window of days may be selected as a set of days that likely includes an ovulation date. The window of days may be selected using a menstrual cycle start date, a menstrual cycle end date, information from previous historical cycles, other suitable information, combinations thereof, and the like. In some variations, when estimating an updated ovulation date of a historical cycle, the temperature shift detection module 212 will use the start date of that historical cycle as well as the start date of the next cycle.

Additionally or alternatively, the ovulation estimation module 208 may include a heart rate estimation module 214 that may be used to estimate an ovulation date using heart rate. A user's sedentary heart rate typically increases shortly before the user's luteal phase begins and rises steadily through ovulation, peaking shortly after ovulation around a beginning of the follicular phase. Thus, a user's sedentary heart rate may be used to predict various portions of a menstrual cycle, or to refine predictions of such portions of a menstrual cycle, such as the onset and end of a fertile window, a period, and/or ovulation. As one non-limiting example, increases in a person's heart rate often start at, near, or shortly after the opening of the fertile window.

For example, in the follicular phase a person's sedentary (e.g., resting) heart rate begins to climb. The person's resting heart rate continues to climb, often (though not necessarily) in a fairly steady fashion, through much of the follicular phase of the menstrual cycle. As the person approaches ovulation, the rate of change of the heart rate generally decreases, although the heart rate itself continues to increase. The fertile window often opens during this increase in heart rate. In many cases, the rise in heart rate for a person during the follicular phase (which stretches from cycle start to ovulation) is fairly sharp and may be, for many people, one-and-a-half to two beats per minute. For most people the follicular phase is less regular than the luteal phase of menstruation, and so tracking changes in heart rate (and the rate of change of a heart rate) may provide the ability to accurately track a duration of the follicular phase as well as predict an onset of ovulation.

By measuring sedentary heart rate, for example through periodic sampling while wearing a wearable device incorporating a heart rate monitor, changes in heart rate (as well as the slope or rate of such changes) may be determined and used to predict various facets of future menstrual cycles, including possible or likely start dates and end dates of fertile windows and periods, as well as the same for ovulation.

Accordingly, the heart rate estimation module 214 may be configured to obtain a set of heart rate data from the user and estimate an ovulation date using the heart rate data, and may estimate this ovulation date using any known technique as would be understand by those of ordinary skill in the art. Because the heart rate estimation module 214 utilizes heart rate data, the ovulation estimation module 208 may use the heart rate estimation module 214 in instances where there is insufficient temperature data to utilize the machine learning module 210 or the temperature shift detection module 212 described previously. The heart rate estimation module 214 may have its own data sufficiency criteria as discussed above.

When the ovulation estimation module 208 estimates the ovulation date of a given menstrual cycle, the system architecture may also use this ovulation date to estimate one or more additional aspects of that cycle. For example, when the ovulation estimation module 208 estimates the ovulation date of an ongoing cycle, the ovulation estimation module 208 may use the estimated ovulation date to estimate the start date of the next period (and thereby estimate the end of the ongoing cycle). In instances where the next period start date was predicted as part of a day one instantiation, this next period start date may replace the original prediction.

Additionally or alternatively, the estimated ovulation date determined by the ovulation estimation module 208 can be provided to a fertile window update module 216. The fertile window update module 216 updates the estimated fertile window in accordance with the determined estimated ovulation date. Depending on which module or modules of the ovulation estimation module 208 are used to determine the estimated ovulation date, this date may be determined from the measured temperature and/or heart rate data.

The ovulation estimation module 208 and fertile window update module 216 may provide any of its estimated dates (e.g., an estimated ovulation date, an estimated fertile window, and/or an estimated next period start date) to the cycle status module 202. The cycle status module 202 may then use these estimated dates when generating a user interface. In some instances, the cycle status module 202 may be configured to display a revised user interface with the new information in response to receiving the updated date(s) from the ovulation estimation module 208 and/or fertile window update module 216.

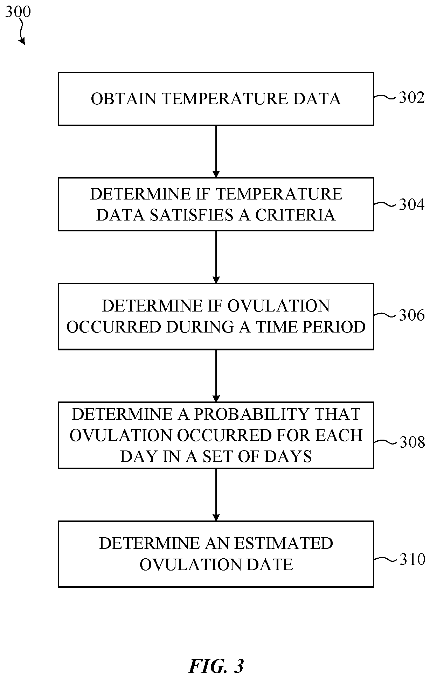

FIG. 3 shows an example process flow 300 for estimating an ovulation date using temperature measurements. The process flow 300 can be performed by the devices described herein including devices with the system architecture 200 described with reference to FIG. 2.

At operation 302, the process 300 can include obtaining a set of temperature data at an electronic device. The temperature data may be based on temperature measurements made by one or more temperature sensors, which can be integrated into the electronic device and/or part of a separate device. A user's temperature may be measured at various times throughout the user's menstrual cycle (including during a preceding menstrual cycle), and these temperature values (along with any associated additional information as discussed above) or information derived therefrom, may be stored at the electronic device and/or remotely (e.g., at a database).

The electronic device may obtain a subset of the stored temperature data to use when performing a particular ovulation estimation. As discussed previously, the electronic device may not attempt to perform an ovulation estimation for a given menstrual cycle (and thereby obtain the subset of temperature data) until certain criteria are met. For example, at operation 302, the electronic device may obtain temperature data for analysis starting on a specific day following the start of a user's menstrual cycle (e.g., their ongoing cycle). When the user logs the start of an ongoing cycle, the electronic device may determine that a threshold amount of time has elapsed from the start date of the menstrual cycle, and obtains the subset of temperature data in response to determining that the threshold amount of time has elapsed.

At operation 304, the process 300 can include determining if the obtained temperature data satisfies one or more criteria (e.g., the data sufficiency criteria as discussed above with respect to the machine learning module 210 of FIG. 2). The electronic device may continue to obtain temperature data until one or more criteria are met. The criteria may be configured to ensure that an ovulation date and/or other menstrual events can be determined with sufficient accuracy depending on the mode in which the electronic device is operating.

At operation 306, the process 300 can include determining if ovulation occurred during a set of days using the obtained set of temperature data. For example, this determination may be performed using the first stage of the machine learning module 210 described above with respect to FIG. 2. The set of days can be determined based on a time period associated with the contained temperature data. In some cases, the set of days can correspond to each day in the time period that temperature data was obtained. In other cases, the set of days may be a subset of the time period associated with the obtained temperature data. For example, the obtained temperature data may correspond to a time period that begins at the start of the menstrual cycle and ends after an initially predicted fertile window. In this example, the set of days may exclude an initial portion of the menstrual cycle (e.g., the period) where ovulation does not occur. For this reason, the set of days may start at a later day in the menstrual cycle (e.g., day 5) and include data from each day until the operation 306 determines that ovulation has occurred. The obtained temperature data from the entire time period may be used to determine if ovulation occurred within a subset of days within the time period.

The obtained temperature data can be input into a classification model and the classification model can output a probability that ovulation occurred during the set of days. If the probability meets a threshold, the process can determine that ovulation occurred during the set of days. In response to the probability meeting the threshold, process 300 can proceed to additional operations, which may analyze the obtained set of temperature data to estimate an ovulation date from the set of days.

If the probability output by the classification model does not meet a threshold, additional temperature data may be collected (e.g., over additional days) and used to generate updated temperature data. The updated temperature data can be input into the classification model. Operation 306 may continue to iteratively collect and input additional temperature data into the classification model until the output probability satisfies the threshold. In some cases, operation 306 may be performed at multiple points over a period of time (e.g., corresponding to the likely ovulation dates) and if the probabilities output by the classification do not meet the threshold, the process 300 may determine that ovulation was not detected. In these cases, the electronic device may use other techniques (e.g., temperature shift detection and/or heart rate measurements to attempt to estimate an ovulation date and/or fertile window).

At operation 308, the process 300 can include determining a set of probabilities that includes a probability that ovulation occurred for each day in the set of days. The obtained temperature data and/or a subset of the obtained temperate data (e.g., associated with the set of days) can be input into a second model that determines a probability that ovulation occurred for each day in the set of days. The second model may be a regression model, such as an LSTM, other artificial neural network model, or other suitable statistical or machine learning model.

At operation 310, the process 300 can include determining an estimated ovulation date using the set of probabilities determined at operation 308. In some cases, the day with the highest probability can be estimated as the ovulation date. In other cases, the probabilities can be analyzed as a set to determine the estimated ovulation date. For example, a mean, median, mode and/or other statistical metric can be used to identify an estimated ovulation date from the set of probabilities.

The estimated ovulation date can be used to determine a fertile window for the user. For example, the fertile window can be estimated to include one or more days preceding the estimated ovulation and one or more days following the estimated ovulation dates. In cases where an initial fertile window was predicated at the period start of the ongoing menstrual cycle, the fertile window determined from the temperature data can be compared to the initial fertile window. If the initial fertile window differs from the newly estimated fertile window (i.e., the fertile window based on the temperature data from the ongoing menstrual cycle), the initial fertile window can be updated.

An indication of the ovulation date and/or the fertile window can be output to the user using the electronic device. The ovulation date and/or fertile window can be displayed to the user using the interface shown in FIG. 4B. In some cases, the electronic device may send an alert, haptic, auditory, or other alert to the user indicating that an ovulation date has been determined and/or the fertile window has been determined or updated.

FIGs. 4A and 4B show example user interfaces 400 displaying estimated fertility and ovulation information. An initial user interface 400a (shown in FIG. 4A) may display initial estimates for a menstrual cycle start day 402 and a fertile window 404, which may be determined using information input by a user, such as start of a period and/or tracked information received from the cycle status module 202. The initial interface 400a may be generated at the start of an ongoing menstrual cycle and be generated in response to a user indicating that their period has started. The initial user interface 400a may display an initial fertile window 404.

The period start date 402 and/or the initial fertile window 404 can be displayed using visual effects to indicate these events. For example, the period start date 402 and/or the initial fertile window 404 can include highlighting, different text, fonts, and/or other effects to indicate the estimated dates for these events in the first user interface 400a.

An updated user interface 400b (shown in FIG. 4B) can be generated in response to determining an estimated ovulation date for an ongoing or previous menstrual cycle, as described herein. The updated user interface 400b may indicate the estimated ovulation date 406 and an updated fertile window 408, which may be determined from obtained temperature data as described herein. While the last day of the updated fertile window 408 is shown in FIG. 4B as coinciding with the estimated ovulation date 406, in other variations the updated fertile window 408 may be selected such that the updated fertile window 408 extends beyond the estimated ovulation date 406 (e.g., one day beyond the estimated ovulation date 406).

Some embodiments may provide a user alert when an ovulation date or fertile window is updated. For example, an electronic device (whether the device calculating the estimates or another associated device) may provide an audible, visual, and/or haptic alert to a user to capture their attention and indicate the update has occurred. Certain embodiments may automatically open and display the calendar with the updated information, as another example. The frequency of these alerts may be limited in some embodiments, such as discussed above.

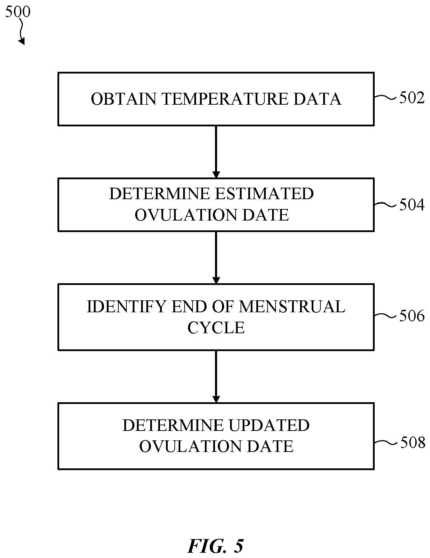

FIG. 5 shows an example process 500 flow for updating an estimated ovulation date and/or fertile window based on additional menstrual cycle data. The process flow 500 can be performed by the devices described herein including devices with the system architecture 200 described with reference to FIG. 2.

At operation 502, the process 500 can include obtaining temperature data for a user at an electronic device. In some cases, obtaining the temperature data may be initiated in response to a start of a menstrual cycle, which may be indicated by user inputs to the electronic device and/or physiological parameters determined at the electronic device. The temperature data may be based on temperature measurements made by one or more temperature sensors. The temperature data may be obtained based on logged and/or detected menstrual events and include data that is measured during the ongoing menstrual cycle (and in some instances, part of a historical cycle preceding the ongoing menstrual cycle). The temperature data may be obtained using the processes as previously described, such as operation 302 from process 300.

At operation 504, the process 500 can include determining an estimated ovulation date using the obtained set of temperature data. In some cases, the ovulation date can be determined using a machine learning process (e.g., using the machine learning module 210 described above with respect to FIG. 2), which may include operations from process 300 described herein. The estimated ovulation date may be determined during an ongoing cycle, for example, shortly after ovulation and when the machine learning process is able to output an ovulation estimate meeting a first criteria (e.g., the output estimated ovulation date is associated with a threshold probability). In other cases, the estimated ovulation date may be output using a temperature shift detection process and/or using heart rate data as described herein.

At operation 506, the process 500 can include identifying an end of the ongoing menstrual cycle. In some cases, the end of the menstrual cycle may be determined from user inputs to the electronic device, such as a user input indicating the start of a period of a next menstrual cycle. The end of the ongoing menstrual cycle can be identified as the day before the start day of the next menstrual cycle.

At operation 508, the process 500 can include determining an updated ovulation date of that menstrual cycle (which has transitioned from the ongoing cycle to a historical cycle) using the end day of the menstrual cycle. The end day of this historical menstrual cycle (alone, or in combination with the start day of the menstrual cycle) can be used to identify a set of days that ovulation may have occurred. In some cases, the set of days can be identified as including days that are a defined number of days from the menstrual cycle end day. For example, a first day of the set of days may be a defined number of days from the identified menstrual cycle end day and the set of days may span a defined number of days.

An estimated ovulation date may be determined from the set of days using a temperature shift process (such as described above with respect to the temperature shift detection module 212 of FIG. 2) that compares temperature information from a first day (or set of days) to temperature information from a second day (or set of days). In one example a six-three processes can be used, where for each day in the set of days, average temperatures from the previous six days are compared to average temperatures from the following three days. The estimated ovulation date can be assigned the day where an increase in the average temperature from the first six days to the second three days increases by a defined threshold. The six-three process is given as one example and other sets of days can be used.

The ovulation date determined using the menstrual cycle end information may be used to update a previously determined ovulation date, which may have been determined at the start of the menstrual cycle and/or during the menstrual cycle (e.g., using the machine learning processes described herein). The updated ovulation date may also be used to update the fertile window, as described herein. In some cases, updates to the ovulation date and/or fertile window based on the menstrual cycle end day may be used to update the user interface and/or to alert the user that the ovulation date has been updated.

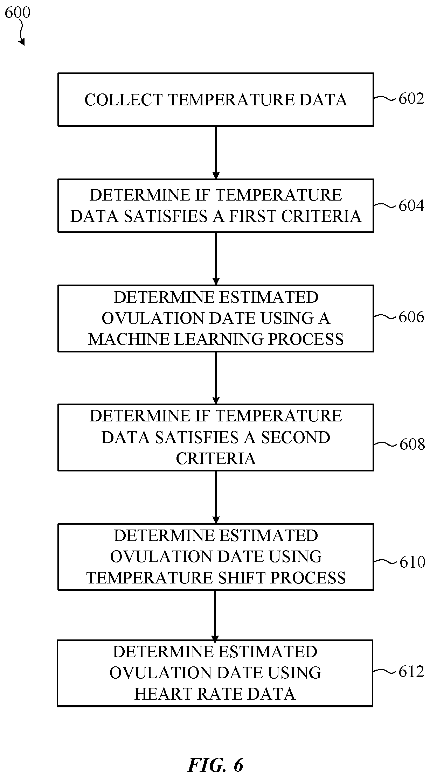

FIG. 6 shows an example process flow 600 for selecting an ovulation estimation process based on the temperature data that has been obtained by the electronic device. Different ovulation estimation processes may have different data sufficiency requirements (e.g., the data sufficiency criteria discussed previously) to be able to estimate an ovulation date with a desired level of accuracy. For example, the machine learning processes described herein may output results with higher accuracy when temperature data is captured over a greater period of time, which can include capturing multiple measurements each day/night. For example, the machine learning processes may perform well on wearable electronic devices that include temperature sensors and are worn by a user throughout the menstrual cycle. Temperature shift processes may be able to estimate an ovulation date using less temperature values per day and/or few days with temperature values, which may be associated with intermittent wearing of one or more temperature sensors. If no or limited temperature data is available, an ovulation date and/or fertile window may be estimated using other physiological measurements including heart rate data as described herein.

At operation 602, the process 600 can include collecting temperature data, which may include the temperature collection processes described herein. The temperature data may be generated from temperature sensors that are worn by a user, for example, temperature sensors that are integrated into a wearable electronic device. Accordingly, the amount and duration of available temperature data may be based on the duration the user wears the watch over the ongoing menstrual cycle.

At operation 604, the process 600 can include determining if a first subset of temperature data satisfies a first criteria. The first criteria can be configured to determine if there is enough temperature data for a machine learning process to output an accurate result. The first criteria may include a minimum temperature data threshold, which can include temperature measurements from a minimum amount of days in the menstrual cycle and/or a minimum amount of temperature measurements each day. Additionally or alternatively, the first criteria can include parameters such as movement parameters, times or day, sleep/awake states, minimum duration for each measurement, and so on.

At operation 606, the process 600 can include determining an estimated ovulation date using a machine learning process in response to the temperature data satisfying the first criteria. The machine learning process can include the processes described herein such as the process 300 described with reference to FIG. 3. The electronic device may be configured to operate in a first mode to determine whether the first subset of temperature data (or any subsequent subsets of temperature data) meet the first criteria and determine an estimated ovulation date using the first subset of temperature data.

At operation 608, the process 600 can include determining if a second subset of temperature data satisfies a second criteria. Operation 608 can be performed as an alternative to or in addition to operation 606. For example, if the temperature data does not satisfy the first criteria at operation 604, then the process 600 can perform operation 608. In other cases, the process 600 may include determining a first estimated ovulation date using operation 606 and then determining whether a second estimated ovulation date can be determined at operation 608.

The second criteria can include a second minimum data threshold that is based on a second data processing algorithm, such as the temperature shift detection process described herein. In some cases, the second data processing algorithm may require less data than the machine learning processes. For example, the temperature shift detection module may require a single temperature measurement from each day. Accordingly, the second criteria may define a minimum data threshold that requires less overall temperature measurements than the first criteria.

At operation 610, the process 600 can include determining an estimated ovulation date using the temperature shift process in response to the temperature data satisfying the second criteria. The temperature shift process can include the processes described herein. In some cases, the temperature shift process may be based on a menstrual cycle end date such as process 500 described with reference to FIG. 5. The electronic device may be configured to operate in a second mode to determine whether the second subset of temperature data (or any subsequent subsets of temperature data) meet the second criteria and determine an estimated ovulation date using the second subset of temperature data.

At operation 612, the process 600 can include determining an estimated ovulation date using heart rate data, as described herein. In some cases, operation 612 may be performed if neither the first criteria nor the second criteria is satisfied for a given subset of temperature data (e.g., a third subset of temperature data). In other cases, operation 612 can be performed in addition to either operation 606 and/or operation 610. In cases where an ovulation date is determined using multiple different processes, the method can be configured to determine an estimated ovulation date using the ovulation date from each of the processes. In some cases, the estimated ovulation date using the output from multiple different processes may weight the outputs of some processes higher (e.g., output from machine learning processes is weighted higher than the output from the heart rate process). Additionally or alternatively, the outputs from the processes can be averaged or analyzed in other ways to generate the estimated ovulation date. The electronic device may be configured to operate in a third mode to determine whether the third subset of temperature data (or any subsequent subsets of temperature data) fails to meet the first and second criteria and determine an estimated ovulation date using the third subset of temperature data.

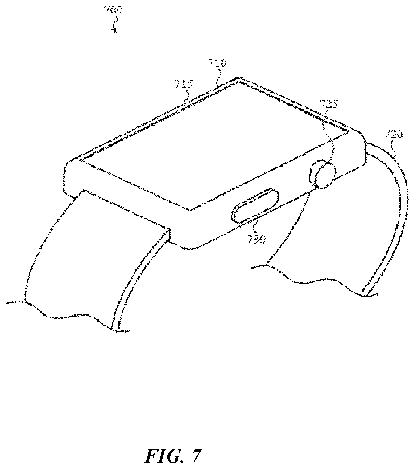

FIG. 7 shows an example wearable device 700 that may execute the methods described herein and/or incorporate the various modules of FIG. 2. Here, the wearable device 700 takes the form of a smart watch although other embodiments may be instantiated in other wearable devices, such as glasses, ear buds, headphones, jewelry, clothing, and so on. Some embodiments may be portable electronic devices that are not necessarily wearable, such as smart phones, tablets, laptop computers, and the like.

In the example of FIG. 7, the wearable device 700 includes a housing 710 coupled to a display 715. The display 715 may show a user interface, such as user interface 400b as discussed previously, to provide information regarding menstrual cycle estimates to a user. These may include estimates regarding a fertile window, period, ovulation, or the like. The display may be touch-sensitive, force-sensitive, or otherwise function as a user input. In this manner the display 715 may accept user data, for example for period tracking or similar.

The wearable device 700 may include one or more other user interfaces, such as a rotatable crown 725, a button 730, and the like. The user may rotate the crown, press or slide the button, or otherwise interact with these devices to provide input. As discussed herein, such input may be used to establish an initial day zero period/fertile window prediction, interact with a module such as the cycle status module 202, or otherwise provide or request information related to menstrual cycle tracking.

The strap 720 may couple the wearable device 700 to the user. This may be especially useful where a heart rate sensor is integrated into or extends through part of the housing 710, for example a rear of the housing. The strap 720 may hold the wearable device in a position relative to the user that permits the heart rate sensor to operate unobtrusively. As one example, the strap may position the wearable device such that the heart rate sensor remains in continuous or near-continuous contact or proximity to the user. This may allow the heart rate sensor to gather heart rate data without requiring any prompts to the user.

As yet another option, a heart rate sensor may be integrated into the crown 725, button 730, display 715, or the strap 720.

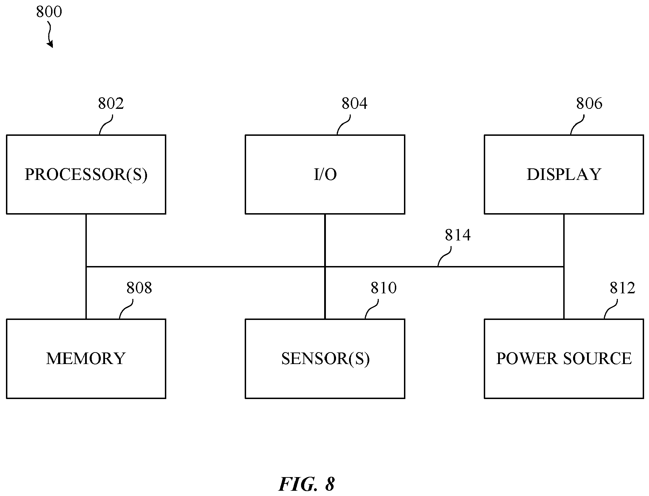

FIG. 8 is an example block diagram of an ovulation monitoring system 800, which can take the form of any of the devices as described with references to FIGs. 1-7. The ovulation monitoring system 800 can include a processor 802, an input/output (I/O) mechanism 804 (e.g., wired or wireless communications interfaces), a display 806, memory 808, sensors 810 (e.g., physiological sensors such as those described herein), and a power source 812 (e.g., a rechargeable battery). The processor 802 can control some or all of the operations of the ovulation monitoring system 800. The processor 802 can communicate, either directly or indirectly, with some or all of the components of the ovulation monitoring system 800. For example, a system bus or other communication mechanism 814 can provide communication between the processor 802, the I/O mechanism 804, the memory 808, the sensors 810, and the power source 812.

The processor 802 can be implemented as any electronic device capable of processing, receiving, or transmitting data or instructions. For example, the processor 802 can be a microprocessor, a central processing unit (CPU), an application-specific integrated circuit (ASIC), a digital signal processor (DSP), or combinations of such devices. As described herein, the term "processor" is meant to encompass a single processor or processing unit, multiple processors, multiple processing units, or other suitable computing element or elements. The processing unit can be programmed to perform the various aspects of the systems described herein.

It should be noted that the components of the ovulation monitoring system 800 can be controlled by multiple processors. For example, select components of the ovulation monitoring system 800 (e.g., a sensor 810) may be controlled by a first processor and other components of the ovulation monitoring system 800 (e.g., the I/O 804) may be controlled by a second processor, where the first and second processors may or may not be in communication with each other.

The I/O device 804 can transmit and/or receive data from a user or another electronic device. An I/O device can transmit electronic signals via a communications network, such as a wireless and/or wired network connection. Examples of wireless and wired network connections include, but are not limited to, cellular, Wi-Fi, Bluetooth, IR, and Ethernet connections. In some cases, the I/O device 804 can communicate with an external electronic device, such as a smartphone, electronic device, or other portable electronic device, as described here.

The ovulation monitoring system may optionally include a display 806 such as a liquid-crystal display (LCD), an organic light emitting diode (OLED) display, a light emitting diode (LED) display, or the like. If the display 806 is an LCD, the display 806 may also include a backlight component that can be controlled to provide variable levels of display brightness. If the display 806 is an OLED or LED type display, the brightness of the display 806 may be controlled by modifying the electrical signals that are provided to display elements. The display 806 may correspond to any of the displays shown or described herein.

The memory 808 can store electronic data that can be used by the ovulation monitoring system 800. For example, the memory 808 can store electrical data or content such as, for example, audio and video files, documents and applications, device settings and user preferences, timing signals, control signals, and data structures or databases. The memory 808 can be configured as any type of memory. By way of example only, the memory 808 can be implemented as random access memory, read-only memory, Flash memory, removable memory, other types of storage elements, or combinations of such devices.

The ovulation monitoring system 800 may also include one or more sensors 810 positioned almost anywhere on the ovulation monitoring system 800. The sensor(s) 810 can be configured to sense one or more types of parameters, such as but not limited to, pressure, light, touch, heat, movement, relative motion, biometric data (e.g., biological parameters), and so on. For example, the sensor(s) 810 may include a heat sensor, a position sensor, a light or optical sensor, an accelerometer, a pressure transducer, a gyroscope, a magnetometer, a health monitoring sensor, and so on. Additionally, the one or more sensors 810 can utilize any suitable sensing technology, including, but not limited to, capacitive, ultrasonic, resistive, optical, ultrasound, piezoelectric, and thermal sensing technology.

The power source 812 can be implemented with any device capable of providing energy to the ovulation monitoring system 800. For example, the power source 812 may be one or more batteries or rechargeable batteries. Additionally or alternatively, the power source 812 can be a power connector or power cord that connects the ovulation monitoring system 800 to another power source, such as a wall outlet.

As described above, one aspect of the present technology is monitoring and managing physiological conditions of a user such as tracking menstrual cycles and the like. The present disclosure contemplates that in some instances this gathered data may include personal information data that uniquely identifies or can be used to contact or locate a specific person. Such personal information data can include demographic data, location-based data, telephone numbers, email addresses, Twitter IDs (or other social media aliases or handles), home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to provide haptic or audiovisual outputs that are tailored to the user. Further, other uses for personal information data that benefit the user are also contemplated by the present disclosure. For instance, health and fitness data may be used to provide insights into a user's general wellness or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining the privacy and security of personal information data. Such policies should be easily accessible by users and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and revised to adhere to applicable laws and standards, including jurisdiction-specific considerations. For instance, in the US, collection of or access to certain health data may be governed by federal and/or state laws, such as the Health Insurance Portability and Accountability Act ("HIPAA"); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of determining spatial parameters, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data at a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, haptic outputs may be provided based on non-personal information data or a bare minimum amount of personal information, such as events or states at the device associated with a user, other non-personal information, or publicly available information.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

Exemplary methods, and electronic devices, are set out in the following items.
1. A method for tracking a menstrual cycle of a user, the method comprising:
   obtaining a first set of temperature data at an electronic device;
   determining that the first set of temperature data meets a first criteria;
   in response to determining that the first set of temperature data meets the first criteria, determining a first probability that ovulation occurred during a first set of days using the first set of temperature data;
   determining that the first probability meets a second criteria;
   in response to determining that the first probability meets the second criteria, determining a set of probabilities;
   selecting an estimated ovulation date from the first set of days using the set of probabilities; and
   displaying an output on the electronic device indicating the estimated ovulation date, wherein:
      the set of probabilities comprises a probability that ovulation occurred for each day in the first set of days.
2. The method of item 1, further comprising:
   obtaining a second set of temperature data at the electronic device;
   determining that the second set of temperature data meets the first criteria;
   in response to determining that the first set of temperature data meets the first criteria, determining a second probability that ovulation occurred during a second set of days using the second set of temperature data; and
   determining that the second probability does not meet the second criteria,
   wherein:
      obtaining the first set of temperature data at the electronic device comprises obtaining the first set of temperature data in response to determining that the second probability does not meet the second criteria.
3. The method of item 1, further comprising:
   determining, after selecting the estimated ovulation date, a duration of the menstrual cycle of the user at the electronic device; and
   determining an updated estimated ovulation date using the determined duration of the menstrual cycle.
4. The method of item 3, wherein determining the updated estimated ovulation date comprises:
   identifying a second set of days using the duration of the menstrual cycle;
   identifying a third set of days using the duration of the menstrual cycle; and
   determining an increase in a temperature of the user from the second set of days to the third set of days.
5. The method of item 1, wherein:
   determining the first probability comprises inputting the first set of temperature data into a classification algorithm; and
   determining the set of probabilities comprises inputting the first set of temperature data into an artificial neural network.
6. The method of item 5, wherein:
   the classification algorithm comprises a random forest classification model; and
   the artificial neural network comprises a long short-term memory artificial neural network.
7. The method of item 1, further comprising:
   determining, prior to obtaining the first set of temperature data, a start date of the menstrual cycle;
   estimating a fertile window based on the determined start of the menstrual cycle; and
   updating the fertile window using the estimated ovulation date.
8. The method of item 7, further comprising, in response to updating the fertile window, causing the electronic device to output a notification to the user indicating the updated fertile window.
9. The method of item 1, further comprising predicting a menstrual cycle end date using the estimated ovulation date.
10. A method for estimating ovulation of a user, the method comprising:
   determining, at an electronic device, a start date of a first menstrual cycle of the user;
   obtaining, following the start date of the first menstrual cycle, a set of temperature data at the electronic device;
   determining, using the set of temperature data, a set of probabilities comprising a corresponding probability that ovulation occurred on each day in the set of days;
   determining an estimated ovulation date of the first menstrual cycle using the set of probabilities;
   displaying a first output on the electronic device based on the estimated ovulation date;
   determining a start date of a second menstrual cycle subsequent to the first menstrual cycle;
   determining an updated ovulation date of the first menstrual cycle using the start date of the second menstrual cycle; and
   displaying a second output on the electronic device based on the updated ovulation date.
11. The method of item 10, further comprising:
   displaying the first output comprises displaying a first indication of a fertile window selected using the estimated ovulation date.
12. The method of item 10, wherein determining the updated ovulation date comprises:
   identifying, at the electronic device, a window of days using the start date of the first menstrual cycle and the start date of the second menstrual cycle;
   for each day in the window of days, comparing a first average temperature for a first number of days preceding the day to a second average temperature for a second number of days following the day to determine a corresponding temperature change; and
   selecting a day in the window of days as the updated ovulation date using corresponding temperature changes determined for the window of days.
13. The method of item 10, comprising:
   inputting the set of temperature data in a classification algorithm; and
   receiving an output from the classification algorithm indicating that ovulation occurred within the set of days.
14. The method of item 13, wherein determining the set of probabilities comprises:
   in response to receiving the output indicating that ovulation occurred, inputting the set of temperature data into an artificial neural network to generate the set of probabilities.
15. The method of item 10, further comprising:
   determining a threshold amount of time has elapsed from the start date of the first menstrual cycle; wherein:
   the set of temperature data is obtained in response to determining that the threshold amount of time has elapsed.
16. An electronic device for tracking menstrual cycles of a user, comprising:
   one or more temperature sensors that measure temperatures of the user;
   a display; and
   a processor configured to collect a set of temperature measurements using the one or more temperature sensors, wherein:
      the processor is configured to operate in a first mode to:
         determine that a first subset of the set of temperature measurements associated with a first set of days meets a first criteria;
         use a first set of operations to determine a first estimated ovulation date for the user using the first subset of the set of temperature measurements, the first set of operations comprising an artificial neural network that outputs a set of probabilities comprising a probability that ovulation occurred for each day of a window of days; and
      the processor is configured in a second mode to:
         determine that a second subset of the set of temperature measurements associated with a second set of days meets a second criteria; and
         use a second set of operations to determine a second estimated ovulation date using the second subset of the set of temperature measurements, a start date of a menstrual cycle, and an end date of the menstrual cycle.
17. The electronic device of item 16, further comprising:
   a heart rate sensor; and
   the processor is configured to operate in a third mode to:
      determine that a third subset of the set of temperature measurements associated with a third set of days fails to meet the first criteria and the second criteria;
      obtain, in response to determining that the third subset of the set of temperature measurements fails to meet the first criteria and the second criteria, a set of heart rate data received from the heart rate sensor; and
      determine a third estimated ovulation date using the set of heart rate data.
18. The electronic device of item 16, wherein:
   determining that the second subset of the set of temperature measurements meets the second criteria comprises determining that the second subset of the set of temperature measurements does not meet the first criteria.
19. The electronic device of item 16, wherein the processor is configured, while operating in the first mode, to:
   inputting the first subset of the set of temperature measurements in a classification algorithm; and
   receiving an output from the classification algorithm indicating that ovulation occurred within the window of days.
20. The electronic device of item 19, wherein the processor is configured, while operating in the first mode, to:
   in response to receiving the output indicating that ovulation occurred, inputting the first subset of the set of temperature measurements into the artificial neural network.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated.

## Claims

1. A method for tracking a menstrual cycle of a user, the method comprising:
obtaining a first set of temperature data at an electronic device;
determining that the first set of temperature data meets a first criteria;
in response to determining that the first set of temperature data meets the first criteria, determining a first probability that ovulation occurred during a first set of days using the first set of temperature data;
determining that the first probability meets a second criteria;
in response to determining that the first probability meets the second criteria, determining a set of probabilities;
selecting an estimated ovulation date from the first set of days using the set of probabilities; and
displaying an output on the electronic device indicating the estimated ovulation date, wherein:
the set of probabilities comprises a probability that ovulation occurred for each day in the first set of days.

2. The method of claim 1, further comprising:
obtaining a second set of temperature data at the electronic device;
determining that the second set of temperature data meets the first criteria;
in response to determining that the first set of temperature data meets the first criteria, determining a second probability that ovulation occurred during a second set of days using the second set of temperature data; and
determining that the second probability does not meet the second criteria,
wherein:
obtaining the first set of temperature data at the electronic device comprises obtaining the first set of temperature data in response to determining that the second probability does not meet the second criteria.

3. The method of claim 1, further comprising:
determining, after selecting the estimated ovulation date, a duration of the menstrual cycle of the user at the electronic device; and
determining an updated estimated ovulation date using the determined duration of the menstrual cycle.

4. The method of claim 3, wherein determining the updated estimated ovulation date comprises:
identifying a second set of days using the duration of the menstrual cycle;
identifying a third set of days using the duration of the menstrual cycle; and
determining an increase in a temperature of the user from the second set of days to the third set of days.

5. The method of claim 1, wherein:
determining the first probability comprises inputting the first set of temperature data into a classification algorithm; and
determining the set of probabilities comprises inputting the first set of temperature data into an artificial neural network.

6. The method of claim 5, wherein:
the classification algorithm comprises a random forest classification model; and
the artificial neural network comprises a long short-term memory artificial neural network.

7. The method of claim 1, further comprising:
determining, prior to obtaining the first set of temperature data, a start date of the menstrual cycle;
estimating a fertile window based on the determined start of the menstrual cycle; and
updating the fertile window using the estimated ovulation date.

8. The method of claim 7, further comprising, in response to updating the fertile window, causing the electronic device to output a notification to the user indicating the updated fertile window.

9. The method of claim 1, further comprising predicting a menstrual cycle end date using the estimated ovulation date.

10. A method for estimating ovulation of a user, the method comprising:
determining, at an electronic device, a start date of a first menstrual cycle of the user;
obtaining, following the start date of the first menstrual cycle, a set of temperature data at the electronic device;
determining, using the set of temperature data, a set of probabilities comprising a corresponding probability that ovulation occurred on each day in the set of days;
determining an estimated ovulation date of the first menstrual cycle using the set of probabilities;
displaying a first output on the electronic device based on the estimated ovulation date;
determining a start date of a second menstrual cycle subsequent to the first menstrual cycle;
determining an updated ovulation date of the first menstrual cycle using the start date of the second menstrual cycle; and
displaying a second output on the electronic device based on the updated ovulation date.

11. The method of claim 10, further comprising:
displaying the first output comprises displaying a first indication of a fertile window selected using the estimated ovulation date.

12. The method of claim 10, wherein determining the updated ovulation date comprises:
identifying, at the electronic device, a window of days using the start date of the first menstrual cycle and the start date of the second menstrual cycle;
for each day in the window of days, comparing a first average temperature for a first number of days preceding the day to a second average temperature for a second number of days following the day to determine a corresponding temperature change; and
selecting a day in the window of days as the updated ovulation date using corresponding temperature changes determined for the window of days.

13. The method of claim 10, comprising:
inputting the set of temperature data in a classification algorithm; and
receiving an output from the classification algorithm indicating that ovulation occurred within the set of days.

14. The method of claim 13, wherein determining the set of probabilities comprises:
in response to receiving the output indicating that ovulation occurred, inputting the set of temperature data into an artificial neural network to generate the set of probabilities.

15. The method of claim 10, further comprising:
determining a threshold amount of time has elapsed from the start date of the first menstrual cycle; wherein:
the set of temperature data is obtained in response to determining that the threshold amount of time has elapsed.

16. An electronic device for tracking menstrual cycles of a user, comprising:
one or more temperature sensors that measure temperatures of the user;
a display; and
a processor configured to collect a set of temperature measurements using the one or more temperature sensors, wherein:
the processor is configured to operate in a first mode to:
determine that a first subset of the set of temperature measurements associated with a first set of days meets a first criteria;
use a first set of operations to determine a first estimated ovulation date for the user using the first subset of the set of temperature measurements, the first set of operations comprising an artificial neural network that outputs a set of probabilities comprising a probability that ovulation occurred for each day of a window of days; and
the processor is configured in a second mode to:
determine that a second subset of the set of temperature measurements associated with a second set of days meets a second criteria; and
use a second set of operations to determine a second estimated ovulation date using the second subset of the set of temperature measurements, a start date of a menstrual cycle, and an end date of the menstrual cycle.

17. The electronic device of claim 16, further comprising:
a heart rate sensor; and
the processor is configured to operate in a third mode to:
determine that a third subset of the set of temperature measurements associated with a third set of days fails to meet the first criteria and the second criteria;
obtain, in response to determining that the third subset of the set of temperature measurements fails to meet the first criteria and the second criteria, a set of heart rate data received from the heart rate sensor; and
determine a third estimated ovulation date using the set of heart rate data.

18. The electronic device of claim 16, wherein:
determining that the second subset of the set of temperature measurements meets the second criteria comprises determining that the second subset of the set of temperature measurements does not meet the first criteria.

19. The electronic device of claim 16, wherein the processor is configured, while operating in the first mode, to:
inputting the first subset of the set of temperature measurements in a classification algorithm; and
receiving an output from the classification algorithm indicating that ovulation occurred within the window of days.

20. The electronic device of claim 19, wherein the processor is configured, while operating in the first mode, to:
in response to receiving the output indicating that ovulation occurred, inputting the first subset of the set of temperature measurements into the artificial neural network.
